# EUROPÄISCHE PATENTSCHRIFT

(11) **EP 0 815 157 B1**
(45) Veröffentlichungstag und Bekanntmachung des Hinweises auf die Patenterteilung: **28.07.1999**
(21) Anmeldenummer: 96907364.2
(22) Anmeldetag: 06.03.1996
(51) Int. Cl.: C08G 63/78, C07C 29/80

(54) **VERFAHREN ZUR AUFARBEITUNG VON DIHYDROXYVERBINDUNGEN ENTHALTENDEN RÜCKSTÄNDEN**
PROCESS FOR PREPARING RESIDUES CONTAINING DIHYDROXY COMPOUNDS
PROCEDE DE PREPARATION DE RESIDUS CONTENANT DES COMPOSES DIHYDROXY

(30) Priorität: 18.03.1995 DE 19509957
(43) Veröffentlichungstag der Anmeldung: 07.01.1998
(73) Patentinhaber: BASF AKTIENGESELLSCHAFT, 67056 Ludwigshafen (DE)
(72) Erfinder: BRAUNE, Peter, D-55234 Erbes-Büdesheim (DE)
(86) Internationale Anmeldenummer: EP9600940
(87) Internationale Veröffentlichungsnummer: WO9629358

(56) Entgegenhaltungen:
- GB-A- 2 045 752
- US-A- 4 110 316
- US-A- 4 239 882
- US-A- 4 499 261
- US-A- 5 236 558

## Beschreibung

Die vorliegende Erfindung betrifft Verfahren zur Aufarbeitung von Dihydroxyverbindungen enthaltenden Rückständen, die bei der Herstellung von Polyestern anfallen, indem man
a) in einer ersten Stufe eine Dicarbonsäure oder deren Ester bzw. esterbildende Derivate mit einem molaren Überschuß einer Dihydroxyverbindung verestert bzw. umestert,
b) in mindestens einer zweiten Stufe das gemäß a) erhaltene Veresterungsprodukt polykondensiert,
c) die bei der Umsetzung gemäß a) und b) anfallenden Brüden einer Behandlung zur Rückgewinnung der Ausgangsstoffe unterwirft.

Zur Herstellung von Polyestern, insbesondere Polyalkylenterephthalaten werden in großem Umfang sogenannte Umesterungs/Polykondensationsverfahren ausgeführt, wobei in einer ersten Stufe eine Veresterung bzw. Umesterung, und in mindestens einer weiteren Stufe die eigentliche Polykondensation vorgenommen wird (vgl. Chemiefasern (Textilindustrie 40 (1992), 1058-1062 und Ullmann's Enzyklopädie der technischen Chemie, 4. Aufl. Band 19, S. 61-88).

Am Beispiel der Herstellung von Polybutylenterephthalat aus Terephthalsäure und Butandiol-1,4 sei dieses Verfahren kurz erläutert.

In einem ersten Reaktionsraum wird Terephthalsäure mit einem molaren Uberschuß, vorzugsweise 50-120 mol-%, insbesondere 70-100 mol-%, Butandiol-1,4 verestert, wobei die veresterte Verbindung in weiteren Schritten der eigentlichen Polykondensation unterworfen wird. Die bei der Veresterung anfallenden Brüden werden in eine Kolonne überführt, in der die leichtsiedende Bestandteile THF/Wasser über Kopf abgetrennt und ein Sumpfprodukt, welches neben überschüssigem Butandiol-1,4 noch geringe Mengen Oligomere, Polymere und Terephthalsäure enthält, erhalten werden.

Das Veresterungsprodukt wird anschließend polykondensiert, wobei die Polykondensation bei kontinuierlichen Verfahren zweckmäßigerweise in mindestens zwei Stufen, einer sogenannten Vorkondensation und Nachkondensation, durchgeführt wird.

Aus wirtschaftlichen Gründen ist es dabei wünschenswert, daß möglichst viele der entstehenden Reaktionsprodukte und im Überschuß vorhandenen Dihydroxyverbindungen weiterbehandelt werden, um z.B. das Butandiol-1,4 zurückzugewinnen und möglichst wenig Abfallstoffe zu erzeugen.

Bei der Herstellung von Polyestern werden üblicherweise die Brüden der Polykondensation gar nicht oder separat aufgearbeitet. Eine Rückführung der Brüden in die Veresterung gegebenenfalls auch nach Abtrennung der Leichtsieder ist hierbei nicht wünschenswert, da derartige Sumpfprodukte einen zu hohen Feststoffgehalt aufweisen. Dieser Feststoffgehalt (oligomere und polymere Bestandteile der Brüden) führt zu einer Verringerung der Produktqualität durch sog. Stippenbildung im Polyester. Unter Stippen versteht man höherschmelzende, gelartige Teilchen, die z.B. bei der Faserherstellung zu Knoten oder Brüchen oder bei der Folienherstellung zu sichtbaren Fehlstellen führen.

Ausgehend von Terephthalsäure besteht zusätzlich das Problem, das für die Veresterung ein wesentlicher größerer Überschuß an Butandiol eingesetzt werden muß, um das Gleichgewicht der Esterreaktion im gewünschten Ausmaß zu verschieben. In den Brüden der Polykondensation, insbesondere der Vorkondensation, ist ein wesentlich größerer Teil des überschüssigen Diols vorhanden als in den Brüden der Veresterung.

Der vorliegenden Erfindung lag daher die Aufgabe zugrunde, die oben geschilderten Nachteile zu verbessern, und die Aufarbeitung der Brüden dahingehend zu verbessern, daß von den darin enthaltenen Dihydroxyverbindungen der größte Anteil in die Veresterung zurückgeführt werden kann, und bezogen auf die gesamte eingesetzte Menge Diol möglichst wenig Diol verloren geht. Gleichzeitig sollte die Produktqualität des Polyesters erhalten bleiben.

Überraschenderweise wurde gefunden, daß diese Aufgabe dadurch gelöst werden kann, daß man
1) die in Stufe a) anfallenden Brüden und einen Teil der Brüden der Stufe b) des Verfahrens in mindestens eine Kolonne A) zusammenführt und die leicht siedenden Bestandteile der Brüden über Kopf abtrennt und das Sumpfprodukt, welches überwiegend die überschüssigen Dihydroxyverbindungen sowie oligomere und polymere Reaktionsprodukte enthält, in die Stufe a) zurückführt und
2) den anderen Teil der Brüden der Stufe b) des Verfahrens in mindestens eine Kolonne B) überführt und die leicht siedenden Bestandteile der Brüden über Kopf abtrennt und das Sumpfprodukt aus der Kolonne austrägt und dieses anschließend einer weiteren Behandlung zur Rückgewinnung der Dihydroxyverbindung unterwirft.

Durch die Aufteilung der Brüden aus der Polykondensation kann auf verfahrenstechnisch einfache Art und Weise eine Rückführung des überschüssigen Diols in die Veresterung erfolgen. Das Verfahren ist dadurch wirtschaftlicher und kostengünstiger, wobei die Produktqualität des Polyesters erhalten bleibt.

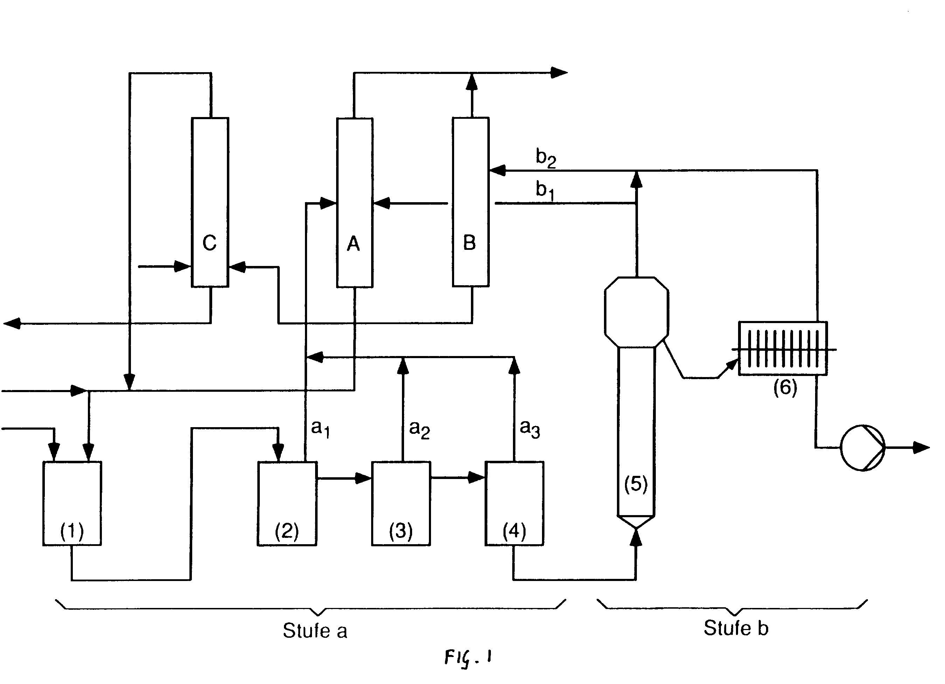

Nachstehend sei das erfindungsgemäße Verfahren wiederum am Beispiel der Herstellung von Polybutylenterephthalat erläutert unter Hinweis auf die Abbildung; es sei jedoch nochmals betont, daß es entsprechend auch für die Herstellung anderer dem Fachmann bekannter Polyester geeignet ist.

Zunächst werden Terephthalsäure und Butandiol-1,4 (letzteres in 150-220 mol-%, vorzugsweise 70-100 mol-% Überschuß) in an sich bekannter Weise bei Temperaturen im Bereich von 150 bis 220°C, und Drücken im Bereich von 0,7 bis 1,5 bar für einen Zeitraum von 30 bis 90, vorzugsweise von 40 bis 70 Minuten miteinander umgesetzt, wobei eine Veresterung stattfindet und entstehendes THF (Tetrahydrofuran) zusammen mit überschüssigem Butandiol (BD) und geringen Mengen oligomerer und polymerer Verbindungen sowie Restmengen Terephthalsäure mit den Brüden (a₁, a₂ und a₃) in eine Kolonne A) überführt werden. Der Ort der Zugabe liegt vorzugsweise in der Mitte oder im unteren Teil der Kolonne.

Die Stufe a) des Verfahrens ist in der Zeichnung in 4 Verfahrensschritte aufgeteilt, dargestellt durch die Rührreaktoren (1) bis (4), welches eine besonders bevorzugte Ausführungsform der Stufe a) darstellt.

Das Veresterungsprodukt der Stufe a) des Verfahrens wird in mindestens einer zweiten Stufe b) polykondensiert. Diese Polykondensation wird in an sich bekannter Weise bei Temperaturen von 240°C bis 270°C und vermindertem Druck von 0,3 bis 200 mbar über einen Zeitraum von 60 bis 200, vorzugsweise 70 bis 180 Minuten durchgeführt. Die Zeichnung zeigt eine besonders bevorzugte Ausführungsform der Stufe b), dargestellt durch die Reaktoren (5) und (6), wobei in (5) eine sog. Vorkondensation und in (6) eine Nachkondensation vorgenommen wird.

Die Brüden der Stufe b) (in der Zeichnung als (b₁) und (b₂) dargestellt), welche überwiegend THF, Wasser, überschüssiges Butandiol und etwas größere Mengen als in den Brüden aus Stufe a) an oligomeren und polymeren Verbindungen sowie Restmengen Terephthalsäure enthalten, werden anteilig in die Kolonnen A) und B) überführt, wobei in Kolonne A) die Brüden aus Stufe a) des Verfahrens bereits vorliegen. Der Ort der Zugabe liegt vorzugsweise in der Mitte oder im unteren Teil der Kolonnen. Wie man diese Anteile aufteilt, versteht sich von selbst, da sich die Aufteilung nach Füllmenge der Kolonnen und benötigten Einsatzstoffen in der Veresterung richtet.

In der Kolonne A) des erfindungsgemäßen Verfahrens wird das leichtsiedende THF/Wasser über Kopf abgetrennt und diese leichtsiedenden Bestandteile anschließend einer Rückgewinnung der Ausgangsstoffe (Trennung THF von Wasser) unterworfen.

Für den Fall der Herstellung von Polyethylenterephthalat bestehen die leichtsiedenden Bestandteile in wesentlichen aus Wasser und Acetaldehyd; bei der Herstellung von PBT aus Dimethylterephthalat aus Methanol und Wasser. Danach führt man das Sumpfprodukt, welches überwiegend die überschüssigen Dihydroxyverbindungen enthält, in die Stufe a) zurück (Reaktor (1)). Den anderen Teil der Brüden der Stufe b) des Verfahrens überführt man in mindestens eine Kolonne B). Je nach Größe der Anlage können die Kolonnen A) und B) in mehrere Kolonnen A₁, A₂ bis Aₙ oder B₁, B₂ bis Bₙ unterteilt werden.

Die leichtsiedenden Bestandteile der Brüden (b₂ aus Stufe b) trennt man über Kopf ab und trägt das Sumpfprodukt aus, welches man anschließend einer weiteren Behandlung zur Rückgewinnung der Dihydroxyverbindung unterwirft. Hierzu trägt man das Sumpfprodukt in eine Kolonne C) aus, unter Abtrennung der Feststoffe.

Bevorzugt wird in der Kolonne C) parallel ein flüssiger Dihydroxyverbindungen enthaltender Rückstand ("Hex"), wie er beispielsweise bei der Destillation von Butandiol-1,4 oder Hexandiol-1,6 anfällt, eingeführt. Die Zusammensetzung des Rückstands unterliegt an sich keiner besonderen Beschränkung, solange die flüssige Form gegeben und keine die Trennung in der Kolonne störenden Verbindungen enthalten sind. Dies ist bei den Rückständen aus der Butandiol bzw. Hexandiol-Destillation in aller Regel der Fall.

Der Ort der Zugabe liegt vorzugsweise in der Mitte oder im unteren Teil der Kolonne, und die Zugabemenge beträgt im allgemeinen 0,03 bis 3 kg pro kg in die Kolonne überführten Sumpfprodukte, vorzugsweise von 0,04 bis 0,1 kg/kg.

Durch die Zugabe dieses Dihydroxyverbindungen enthaltenden Rückstandes bleibt das Sumpfprodukt in der Kolonne C) flüssig bzw. förderbar, und es kann daher auf verfahrenstechnisch einfache Weise die Dihydroxyverbindung über Kopf und ein wiederum flüssiges bzw. förderbares Sumpfprodukt am Boden dieser Kolonne erhalten werden, welches einfach der Verbrennung zugeführt werden kann.

Die Dihydroxyverbindung führt man anschließend in die Veresterung (Stufe a) zurück.

Die Vorteile des erfindungsgemäßen Verfahrens liegen darin, daß möglichst große Anteile der Brüden auf einfache Weise in die Veresterung zurückgeführt werden können. Dies bringt nochmals eine Vereinfachung und Kostenersparnis mit sich, ohne daß die Produktqualität beeinträchtigt wird.

### Beispiel

In einer Rührkesselkaskade wurden 306 kg/h Terephthalsäure, 332 kg/h Butandiol (molares Verhältnis 1 : 2) und 30 ppm Ti als Tetrabutylorthotitanat, bezogen auf 1 kg PBT (Katalysator) bei einer Temperatur von 230°C und einem Druck von 1 bar und einer mittleren Verweilzeit von 205 Minuten verestert. Die bei der Veresterung freiwerdenden Brüden wurden kontinuierlich in eine Kolonne A) überführt. Das Umesterungsprodukt wurde mit einem Umsatz von >95 % in die Polykondensationsstufe b) überführt. Die Brüden der Stufe b) wurden kontinuierlich in 2 Kolonnen überführt, so daß 56 % in Kolonne A) mit den Brüden aus der Stufe a) zusammengeführt wurden und 44 % in eine Kolonne B) überführt wurden.

### Aufarbeitung Kolonne A)

Aus den Brüden aus Stufe a) und b) wurden zunächst THF und H₂O über Kopf abgetrennt und das Sumpfprodukt mit einem Durchsatz von 167 kg/h in die Veresterung zurückgeführt.

### Aufarbeitung Kolonne B)

Aus dem Teil Brüden aus Stufe b) des Verfahrens wurden zunächst THF und H₂O (Leichtsieder) über Kopf abgetrennt und das Sumpfprodukt mit einem Durchsatz von 58 kg/h unter Abtrennung der Feststoffe in eine Kolonne C) überführt, in deren Mitte 4 kg/h eines bei der Butandiol-1,4-Destillation angefallenen flüssigen Rückstands (enthielt als Hauptbestandteile Butandiol-1,4, 2-Methylpentandiol-1,5, Hexandiol-1,6, 2-Methylhexandiol-1,6, Pentantriol-1,2,5) zugegeben wurden.

Butandiol wurde am Kopf der Kolonne C) und ein flüssiges Sumpfprodukt am Boden der Kolonne ausgetragen. Das Sumpfprodukt wurde über eine Rohrleitung einer Verbrennungseinrichtung zugeführt.

Durch die erfindungsgemäße Aufarbeitung wurden 56 mol-% des 100%igen molaren Überschusses an Butandiol nach der Befreiung von Leichtsiedern in die Stufe a) zurückgeführt. 44 mol-% wurden destillativ aufgearbeitet. Diese mol-% Angaben beziehen sich auf den BD-Überschuß nach Abtrennung der THF-Anteils. 20 mol-% des sog. Äquivalent-Butandiolanteils werden üblicherweise in THF und Wasser durch die Reaktion umgewandelt (Äquivalent-Butandiol, bedeutet den theoretischen molaren Anteil, der für die Umsetzung benötigt wird). Das der Verbrennungseinheit zugeführte Sumpfprodukt bestand zu 63 % aus dem Rückstand der Butandiol-Destillation ("Hex"), zu 18,5 % aus festen Rückständen (oligomere/polymere Anteile) und zu 18,5 % aus Butandiol aus der Umsetzung. Diese 18,5 % Butandiol des Rückstandes waren lediglich 2 % der gesamten der Kolonne C) zugeführten Butandiolmenge. Somit gingen insgesamt 0,35 % Butandiol verloren, bezogen auf das bei der Veresterung eingesetzte Butandiol (TPA: BD= 1 : 2 mol).

## Patentansprüche

1. Verfahren zur Aufarbeitung von Dihydroxyverbindungen enthaltenden Rückständen, die bei der Herstellung von Polyestern anfallen, indem man
a) in einer ersten Stufe eine Dicarbonsäure oder deren Ester bzw. esterbildende Derivate mit einem molaren Überschuß einer Dihydroxyverbindung verestert bzw. umestert,
b) in mindestens einer zweiten Stufe das gemäß a) erhaltene Veresterungsprodukt polykondensiert,
c) die bei der Umsetzung gemäß a) und b) anfallenden Brüden einer Behandlung zur Rückgewinnung der Ausgangsstoffe unterwirft,
dadurch gekennzeichnet, daß man
1) die in Stufe a) anfallenden Brüden und einen Teil der Brüden der Stufe b) des Verfahrens in mindestens eine Kolonne A) zusammenführt und die leicht siedenden Bestandteile der Brüden über Kopf abtrennt und das Sumpfprodukt, welches überwiegend die überschüssigen Dihydroxyverbindungen sowie oligomere und polymere Reaktionsprodukte enthält, in die Stufe a) zurückführt und
2) den anderen Teil der Brüden der Stufe b) des Verfahrens in mindestens eine Kolonne B) überführt und die leicht siedenden Bestandteile der Brüden über Kopf abtrennt und das Sumpfprodukt aus der Kolonne austrägt und dieses anschließend einer weiteren Behandlung zur Rückgewinnung der Dihydroxyverbindung unterwirft.

2. Verfahren nach Anspruch 1, dadurch gekennzeichnet, daß die leicht siedenden Bestandteile der Brüden im wesentlichen aus Wasser, Tetrahydrofuran, Acetaldehyd und Methanol bestehen.

3. Verfahren nach den Ansprüchen 1 bis 2, dadurch gekennzeichnet, daß man das Sumpfprodukt der Kolonne B) unter Abtrennung der Feststoffe in eine Kolonne C) ausgeträgt, einen flüssigen, Dihydroxyverbindungen enthaltenden Rückstand zugibt und anschließend eine Behandlung zur Rückgewinnung der Dihydroxyverbindung durchführt.

## Claims

1. A process for working up dihydroxy compound residues from the preparation of polyesters by
a) a first step of (trans)esterifying a dicarboxylic acid or its esters or ester-forming derivatives with a molar excess of a dihydroxy compound,
b) at least one second step of polycondensing the esterification product of a), and
c) subjecting the vapors from the reaction of a) and b) to a treatment for recovering the starting materials,
which comprises
1) combining the vapors of step a) and some of the vapors of step b) of the process in at least one column A) and removing the low boiling constituents of the vapors overhead and recycling the bottom product, which predominantly contains the excess dihydroxy compounds and also oligomeric and polymeric reaction products, into step a), and
2) transferring the other part of the vapors of step b) of the process into at least one column B) and removing the low boiling constituents of the vapors overhead and discharging the bottom product from the column and subsequently subjecting it to a further treatment for recovering the dihydroxy compound.

2. A process as claimed in claim 1 wherein the low boiling constituents of the vapors consist essentially of water, tetrahydrofuran, acetaldehyde and methanol.

3. A process as claimed in claim 1 or 2 wherein the bottom product of column B) is discharged into a column C) with removal of the solids, a liquid dihydroxy compound residue is added, and subsequently a treatment for recovering the dihydroxy compound is carried out.

## Revendications

1. Procédé de traitement de résidus contenant des composés dihydroxy, qui sont obtenus lors de la fabrication de polyesters, dans lequel
a) dans une première étape, on estérifie ou transestérifie un acide dicarboxylique ou ses esters ou respectivement dérivés estérogènes avec un excès molaire d'un composé dihydroxy,
b) dans au moins une deuxième étape, on polycondense le produit d'estérification obtenu selon a),
c) on soumet les vapeurs obtenues lors de la réaction selon a) et b) à un traitement pour la récupération des matières de départ,
caractérisé en ce que
1) on réunit les vapeurs formées dans l'étape a) et une partie des vapeurs de l'étape b) du procédé dans au moins une colonne A), on isole par la tête les composants à faible point d'ébullition des vapeurs et on recycle dans l'étape a) le produit de fond de cuve, qui contient d'une manière prépondérante les composés dihydroxy excédentaires ainsi que des produits réactionnels oligomères et polymères, et
2) on transfère l'autre partie des vapeurs de l'étape b) du procédé dans au moins une colonne B) et on isole par la tête les composants à faible point d'ébullition des vapeurs et on évacue de la colonne le produit de fond de cuve, en soumettant ensuite ce dernier à un traitement ultérieur pour la récupération du composé dihydroxy.

2. Procédé suivant la revendication 1, caractérisé en ce que les composants à faible point d'ébullition des vapeurs sont essentiellement constitués d'eau, de tétrahydrofuranne, d'acétaldéhyde et de méthanol.

3. Procédé suivant l'une des revendications 1 et 2, caractérisé en ce qu'on évacue le produit de fond de cuve de la colonne B) avec séparation des matières solides dans une colonne C), on ajoute un résidu liquide contenant des composés dihydroxy et ensuite on effectue un traitement pour la récupération du composé dihydroxy.
